# EUROPEAN PATENT SPECIFICATION

(11) **EP 3 073 921 B1**
(45) Date of publication and mention of the grant of the patent: **31.01.2018**
(21) Application number: 13818721.6
(22) Date of filing: 18.12.2013
(51) Int. Cl.: A61B 5/15, A61B 5/145, A61B 5/1455, A61B 5/1468, A61B 5/151, A61B 5/157

(54) **BODY FLUID GUIDING CAPILLARY STRUCTURE FOR SELF-CONTAINED TEST UNIT**
KÖRPERFLÜSSIGKEITSLEITENDE KAPILLARSTRUKTUR FÜR IN SICH GESCHLOSSENE PRÜFEINHEIT
STRUCTURE CAPILLAIRE DE GUIDAGE DU FLUIDE CORPOREL POUR UNITÉ DE TEST AUTONOME

(30) Priority: 19.12.2012 GB 201222925
(43) Date of publication of application: 05.10.2016
(73) Proprietor: Polymer Technology Systems, Inc., Indianapolis, IN 46268 (US)
(72) Inventor: RITTMANNSBERGER, Franz, A-8720 Knittelfeld (AT); HAFELLNER, Reinhard, A-9724 Spielberg (AT)
(74) Representative: Zinkler, Franz
(86) International application number: PCT/EP2013/077090
(87) International publication number: WO 2014/096001

(56) References cited:
- EP-A1- 1 541 087
- WO-A1-02/100278
- WO-A2-02/100254
- US-A- 4 627 445
- US-A1- 2009 105 613
- US-B2- 7 396 334
- US-B2- 7 846 110

## Description

The invention relates to a self contained test unit for testing body fluid.

Moreover, the invention relates to an arrangement for testing body fluid.

Furthermore, the invention relates to a method of testing body fluid by using a self contained test unit.

Diabetis patients need a simple system of measuring glucose level several times a day. Background art of this technical field is found in DE 101 34 650, EP 2,277,442, or EP 1,486,766.

US 7,846,110 discloses a self contained test unit for testing body fluid which comprises a body member and a support member. The support member is moveable with respect to the body member between a first position and a second position. The support member includes a body part receiving surface for receiving a patient's body part. A lancet is carried by the body member and includes a lancet tip capable of piercing the skin of a patient to produce fluid flow. A test member is capable of interacting with body fluid to aid in the determination of information about body fluid components. A capillary member is capable of guiding fluid flow to the test member. A pressure cup is capable of exerting pressure on a body part to foster fluid flow out of a lanced site and into the capillary, and a calibration member is provided for containing information for facilitating calibration of the test unit.

However, although the system of US 7,846,110 works fine, it may still be a challenge under undesired circumstances to reliably achieve flow of body fluid, particularly when the volume of the body fluid to be transported is very small, from the body part receiving surface via the capillary member to the test member.

It is an object of the invention to accurately guide a flow of body fluid from a body part receiving surface to a test member of a test unit, particularly when the volume of the body fluid to be transported is very small.

In order to achieve the object defined above, a self contained test unit for testing body fluid, an arrangement for testing body fluid, and a method of testing body fluid by using a self contained test unit according to the independent claims are provided.

According to an exemplary embodiment of the invention, a self contained test unit for testing body fluid is provided, the test unit comprising a body part receiving surface for receiving a patient's body part (particularly configured for receiving a tip of a finger of a patient), a movably mounted (for instance metallic) lancet including a lancet tip configured for piercing the skin of the patient to produce flow of body fluid (particularly blood) when the patient's body part is received at the body part receiving surface and when the lancet has been moved so that the lancet tip is in interaction with the patient's body part, and a capillary member having a first recess configured for receiving the lancet and having a second recess configured for guiding the body fluid from the body part receiving surface to a test member for interacting with the body fluid to aid in the determination of information about body fluid components, wherein the second recess has a larger surface area (particularly the surface area of all walls of the capillary member delimiting the respective recess) to volume (particularly the entire volume of the respective recess from its inlet juxtaposed to the body part receiving surface to its outlet juxtaposed to the test member) ratio than the first recess.

According to another exemplary embodiment of the invention, an arrangement for testing body fluid is provided, the arrangement comprising a self contained test unit having the above-mentioned features, and a control device having a test unit reception configured for detachably receiving the test unit and a detection unit configured for detecting the information about body fluid components by determining an impact of the interaction between the body fluid and the test member.

According to still another exemplary embodiment of the invention, a method of testing body fluid by using a self contained test unit is provided, wherein the method comprises receiving a patient's body part on a body part receiving surface of the test unit, moving a lancet, including a lancet tip, of the test unit through a first recess of a capillary member of the test unit so that the lancet tip is in interaction with the patient's body part for piercing the skin of the patient to produce flow of body fluid, and bringing a test member of the test unit in interaction with the body fluid by guiding the body fluid from the body part receiving surface through a second recess of the capillary member to the test member to aid in the determination of information about body fluid components, wherein the second recess has a larger surface area to volume ratio than the first recess.

The term "surface area to volume ratio" of a recess can particularly denote a value which is obtained by dividing a surface area of all walls delimiting the recess by the volume of the recess, i.e. the volume which is delimited between the inlet and the outlet of the recess. In case of an open recess which for instance has an open inlet and an open outlet, only the surface area of walls delimiting the recess are to be counted for estimating the surface area, since only the surface area of such walls contributes to surface adhesion of the body fluid in the respective recess. The expression that the second recess has a larger surface area to volume ratio than the first recess is, in technical terms, equivalent to the formulation that the capillary forces keeping the body fluid within the second recess are larger than capillary forces the body fluid would experience in the first recess. In case of an integral recess including the first recess and the second recess, the first recess is defined by the part of the integral recess within which the lancet moves. The second recess is then the remainder of the integral recess, i.e. the remaining part of the integral recess within which the lancet cannot move.

According to an exemplary embodiment of the invention, a test unit for testing a body fluid is provided in which the transport of the body fluid from a position of a lancet tip piercing the patient so as to produce flow of body fluid towards the test member interacting with the body fluid to derive the information of the test is simplified and rendered accurate and reproducible. This guiding of the body fluid exclusively along the desired second recess is a physical consequence of the configuration of the first and second recesses so that capillary forces keep the body fluid within and along the second recess in view of its larger surface area to volume ratio, as compared to the first recess. By taking this measure, it is possible even for relatively unskilled users to perform a successful and reproducible test which is not disturbed or rendered impossible by an uncontrolled or undesired flow of body fluid transport along a capillary. This also holds when the volume of the body fluid (particularly blood) to be transported is very small, particularly smaller than 500 nl, more particularly smaller than 300 nl.

Hence, an embodiment provides a capillary geometry for transport of body fluid such as whole blood to a sensor (for instance a photometric or electrochemical sensor) on a test unit which comprises a reactive component (such as a foil with reactive chemistry film), a capillary component (defining geometry of blood flow), and a lancet component (a component for penetrating the skin). In an embodiment, the transport capillary channel is designed that the body fluid is intended to flow in an edge area and not in the whole cross section (so that a smaller amount of body fluid such as blood volume is sufficient for carrying out the test). The edge is the narrower part of the channel and has edge-zones with a sufficiently small radius (for instance below 0.2 mm). The capillary geometry may be produced in an injection moulding process.

In the following, further exemplary embodiments of the self contained test unit for testing body fluid, the arrangement for testing body fluid, and the method of testing body fluid by using a self contained test unit will be explained.

In an embodiment, a volume of the second recess is less than approximately 400 nl, particularly less than approximately 200 nl. Such small volumes of body fluid such as blood can be handled with the test unit in view of the geometry of the second recess which accumulates the body fluid in a small area and reliably forwards it to the test member. Thus, the test unit is very convenient in use.

In an embodiment, the first recess and the second recess are configured as an integral common recess. The second recess may particularly be a tapering extension extending from the first recess. By designing the first recess and the second recess as a continuous connected void, a simple and compact construction of the capillary can be obtained. Particularly when manufacturing the test unit by injection moulding, the formation of the two recesses to one combined recess is advantageous. The second recess can be free of narrow pockets so as to maintain a sufficiently large space for moving the lancet through the first recess and for preventing undesired flow of body fluid through this recess. In contrast to this, the second recess may be a narrow extension of the first recess which may end in a narrow corner in which the body fluid is accumulated so as to concentrate the flow of body fluid into this volume.

In an embodiment, a cross-sectional area of the first recess, in a viewing direction perpendicular to the flow direction of the body fluid from the body part receiving surface to the test member, is larger than a cross-sectional area of the second recess, in a viewing direction perpendicular to the flow direction of the body fluid from the body part receiving surface to the test member. Thus, the cross-sectional area of the lancet guided along the first recess may be larger than the cross-sectional area of the body fluid guiding second recess. Therefore, both tasks may be performed in the different recesses without undesired interaction, since in view of the larger cross-sectional area of the first recess, the high surface adhesion of aqueous body fluids prevents the body fluid from flowing into the first recess. In other words, the body fluid will be forced to flow into the second recess.

Particularly, from a geometrical point of view, the geometric ratio of perimeter to cross-sectional area of the first recess shall be sufficiently large, more particularly larger than for the second recess. In a corresponding physical picture, the contact line at the flow front shall be sufficiently large. The fluid has the tendency to wet the surface, which increases the dimension of the contact line and consequently increases the net force for the capillary effect. An opposing force is the surface tension on the free surface of the fluid (having the tendency to assume a sphere shape). The free surface shall be as small as possible.

In an embodiment, the second recess has a surface area to volume ratio being at least about twice, particularly at least about five times, of a surface area to volume ratio of the first recess. It has turned out that such significant differences regarding the surface area to volume ratios results in a clear functional separation of the two recesses, so that the body fluid flows only in the second recess and the lancet is movable only in the first recess.

In an embodiment, the first recess has a basically circular shape in a viewing direction perpendicular to the flow direction of the body fluid from the body part receiving surface to the test member. A circular shape prevents narrow edges so that body fluid can be safely prevented from flowing into the first recess. Furthermore, the provision of a circular cross-sectional shape allows to guide a circular cylindrical lancet along the first recess with form adaptation.

In an embodiment, the second recess is a basically triangular structure in a viewing direction perpendicular to the flow direction of the body fluid from the body part receiving surface to the test member, particularly is a V-shaped notch. A triangle (particularly with two equal side lengths for symmetry purposes) as cross-sectional geometric form of the second recess promotes an accumulation of the body fluid in the narrow corner or edge thereof, because here the local surface area to volume ratio is very high. Thus, the second recess may have a beak-shaped appearance.

In an embodiment, the first recess corresponds to the void volume within the capillary member within which the lancet is movable. Correspondingly, the second recess corresponds to the void volume within the capillary member within which the lancet is not movable, or in accordance with which is free for the body fluid to move along the capillary member in any operation state of the test unit.

In an embodiment, the triangular structure has an angle at an end opposing the first recess in a range between about 20° and about 120°, particularly in a range between about 30° and about 70°. With angles being significantly lower than 20°, it may become difficult or cumbersome to produce the test unit by injection moulding. For angles being significantly larger than 120°, the influence of capillary effects is quantitatively smaller and the desired impact of surface adhesion becomes smaller as well.

In an embodiment, the triangular structure has (for instance two equal) side lengths extending (particularly in a tapering way) from the first recess towards a tip as an end of the second recess in a range between about 0.01 mm and about 2 mm, particularly in a range between about 0.1 mm and about 0.1 mm. These dimensions ensure a transport of a sufficiently large amount of body fluid along the capillary member, while at the same time keeping the surface area to volume ratio sufficiently large.

For instance, the diameter of the lancet can be 0.4 mm. The first recess may have a diameter of 0.5 mm. The side length of the triangle may be in the order of magnitude of the diameter.

In an embodiment, at least the capillary member is made of a plastic material, particularly of polycarbonate, polystyrene, polystyrene, polybutylene terephthalate, poly(methyl methacrylate, polyvinyl chloride, or polypropylene. Furthermore, such materials are compatible with injection moulding manufacturing procedures.

The choice of an appropriate surface material in the fluid guiding recess may be based on the following considerations: It is advantageous to have a material being properly wettable by the body fluid. This may be promoted by a corresponding surface activation (physical or chemical or coating). For instance, polypropylene is cost efficient and has, after coating, suitable properties for the capillary member.

In an embodiment, at least walls of the capillary member delimiting the second recess (particularly walls of the capillary member delimiting the first recess and the second recess) are surface-configured to provide a contact angle of less than about 90°, particularly of less than about 60°, between a wall delimiting the second recess and the body fluid partly wetting this wall. Particularly, these walls may be coated with a hydrophilic coating, may be chemically surface activated, or may have a physical surface structure. However, also a chemical surface activation (for instance by a plasma treatment) or a physical surface structure (such as a nanostructure) are possible for promoting the fluid transport properties. Such a coating of the walls with a thin surface layer may ensure a proper wetting of the wall by the body fluid, thereby promoting flow of the body fluid within and along the second recess.

In an embodiment, the cross-sectional shape of the first recess and/or of the second recess, in a viewing direction perpendicular to the flow direction of the body fluid, remains constant along a first part (of for instance more than 50%, particularly of more than 80%, of the path length) of a path but does not remain constant along a (remaining) second part of the path. This ensures a basically undisturbed constant and continuous flow of the body fluid along the entire capillary member, while at the same time providing a guide structure for moving the lancet.

In another embodiment, the cross-sectional shape of the second recess, in a viewing direction perpendicular to the flow direction of the body fluid, widens up, particularly continuously widens up, along a flow path of the body fluid along the second recess. This promotes a reliable lateral transfer of the body fluid to the test member.

In an embodiment, at least the capillary member is an injection moulded component. Injection molding is a manufacturing process for producing parts from both thermoplastic and thermosetting plastic or other materials including metals, glasses, elastomers and confections. Material is fed into a heated barrel, mixed, and forced into a mold cavity where it cools and hardens to the configuration of the cavity. By manufacturing the test unit at least partially by injection moulding, it can be manufactured at reasonable costs.

In an embodiment, the capillary member has a basically sharp edge at its end juxtaposing the test member. At the contact area of injection molded capillary geometry and chemistry, the geometry may hence have a sharp edge (mould can be produced to achieve this), for instance with a maximum radius of 0.05 mm. Such a sharp edge ensures a proper fluid flow from the end of the capillary member onto the test member. Thus, the complete process of body fluid extraction via capillary transport to the triggering of an interaction with the test member is possible in a failure-robust way.

In an embodiment, the basically sharp edge has a V-shape, an L-shape, or a curvature with a radius of less than about 0.1 mm, particularly of less than about 0.05 mm. Such dimensions have turned out to ensure a proper body fluid transport from the lower end of the capillary member onto the test member and still provides a relatively sharp transition.

In an embodiment, the capillary member has a protrusion at its end juxtaposing the body part receiving surface, wherein the first recess and the second recess extend through the protrusion. Such a local projection has the advantage that the fluidic coupling between the body part and the capillary member is improved, since, due to capillary forces and surface adhesion of the body fluid, the body fluid is forced to enter the recess within the capillary member when contacting the protrusion.

In an embodiment, the first recess extends through a central portion of the protrusion and the second recess extends through a lateral portion of the protrusion. Then, the lancet can move to a central portion of the body part to pierce it, whereas the body fluid flow may be shifted to a lateral or side position.

In an embodiment, an upper surface of the protrusion is slanted in such a way that the second recess extends through a portion of the protrusion at an upper end of the slanted upper surface. The capillary entrance has two extending areas which are narrower to the skin, which guarantees first contact to blood after lancing and leads the blood to the transport capillary. Such a slanting of the protrusion allows to form a local maximum at the position at which the extracted body fluid flows into the second recess of the capillary member. Therefore, the guided body fluid flow along the entire capillary member is further refined.

In an embodiment, an upper surface of the protrusion has two tips between which the second recess extends through the protrusion. Also by taking this measure, the fluid flow can be accurately defined and rendered reproducible.

In an embodiment, the test unit comprises a cap, preferably integrally formed with a body member of the test unit, more preferably pivotably connected to a body member of the test unit by an integral hinge (which may be a locally thinned portion of a plastic member), the cap being configured to be selectively opened (by pivoting in a first direction) for exposing the body part receiving surface or closed (by pivoting in a second direction opposing the first direction) for covering the body part receiving surface. By covering the test unit in a default state, the inner part of the test unit may remain basically protected before first use. A cap having an integral hinge connected to a body member can be manufactured by injection moulding.

In an embodiment, the test unit comprises at least one ventilation hole (particularly an arrangement of a plurality of circumferentially distributed ventilation holes) for providing an air communication between the body part receiving surface to be covered by the cap and another part (for instance a bottom) of the test unit being exposed to atmospheric pressure. Closing the test unit by pivoting the cap onto the body member can cause a sudden compression of air in an interior of the test unit. By however forming one or more ventilation holes within the test body, even in case of such a sudden closure of the test unit, undesired pressure pulses may be prevented.

In an embodiment, the test unit comprises a body member and a support member moveable with respect to the body member between different positions, the support member accommodating the lancet. Triggering a relative motion between body member and support member may result in a movement of the lancet along the first recess of the capillary member for triggering the piercing.

In an embodiment, the support member comprises a control device attachment section configured for being attached to a test unit reception of a control device. An actual fastening of the test unit at the control device may be performed by a bayonet closure, which may be located at the bottom of the light guide.

Guiding structures may be provided on the capillary component and the light guide component. Guide structures allow to guide the light guide in the capillary member parallel and without the risk of tilting. Such guide structures prevent a tilting to protect the lancet from becoming stuck.

In an embodiment, the test unit comprises a pressure cup for exerting pressure on the body part to foster fluid flow out of a lanced site and into the second recess of the capillary member. Such a pressure cup may surround the capillary entrance to force blood to flow outside of the skin to the capillary entrance (the test unit may be designed so that the force for activation is also used for the pressure cup). The pressure cup may form a circumferential rim. When a user presses his or her finger onto the rim, the central portion of the finger within the rim will be compressed and will be forced towards a position to which a lancet may be moved for piercing. Such a compressed portion of the finger brings the tissue into a shape in which the piercing is possible without significant pain. Furthermore, the pressure applied to the tissue promotes the flow of body fluid into the capillary member.

In an embodiment, the pressure cup surrounds an upper end, particularly an upper protrusion, of the capillary member. Therefore, direct flow of body fluid from the body part compressed by the pressure cup onto the capillary member may be ensured.

In an embodiment, the test unit is configured so that exerting pressure on the pressure cup simultaneously moves the support member with respect to the body member to thereby move the lancet in such a way that the lancet tip pierces the skin of the body part upon exerting pressure on the pressure cup. By such a motion mechanism, a user may trigger the entire body fluid extraction procedure with a single motion.

In an embodiment, the test unit is configured so that the lancet is further moved to be deactivatedly accommodated within an interior of the test unit upon continued exertion of pressure after the piercing. After the piercing, the lancet may be retracted towards an idle mode or an inactive position at which any subsequent forward motion of the sharp tip of the lancet towards the finger of the patient is prevented. Therefore, the described mechanism can serve as a safety mechanism.

In an embodiment, the test unit is configured as a self contained disposable test unit which can only be used once. The test unit can hence be designed as a safety product in which only a single contact of the lancet with the skin is possible. Hence, upon deactivating the lancet tip after first use, any second use of the test unit under non-sterile conditions is disabled.

In an embodiment, the test unit comprises the test member configured for interacting with the body fluid to aid in the determination of information about body fluid components (such as a glucose level in blood). In such an embodiment, the optional test member forms part of the test unit.

In an embodiment, the test member is an optically transparent ring which comprises a substance which is configured for chemically reacting with the body fluid and has a central recess being traversable by the lancet. Such a substance may be a chemical configured for chemically reacting with glucose in patient's blood so as to allow to determine the patient's glucose level based on the result of the chemical reaction.

In an embodiment, the test member is configured for chemically reacting with the body fluid to thereby create a reaction product being detectable, particularly quantitatively detectable, photometrically. The chemistry film can hence be based on a photometric principle. Thus, when the body fluid comes in interaction with the test member, the photometric properties of the test member change which changes can be detected by a camera.

In an embodiment, the test unit comprises an optical member, particularly a light guide and/or a focusing lens, being configured for optically manipulating, particularly for guiding or focusing, electromagnetic radiation (such as visible light) from the test member and/or a calibration member towards a detection unit of a control device on which the test unit is mounted. Such a focusing lens may focus light onto a detector to improve the accuracy of the detection. Such a light guide may guide light along a controlled path through a part of the test unit.

In an embodiment, the test unit comprises a calibration member configured for containing information for calibration of the test unit. By calibrating the measurement, the reliability of the results can be increased.

In an embodiment, the calibration member is an optically opaque or transparent ring having a central recess being traversable by the lancet and containing the information in the form of markers being arranged circumferentially around the central recess. Thus, basically the entire area of the calibration member can be used either as a guide for the lancet or to carry calibration information.

The central recess of the calibration member should be larger than the central recess of the test member and the calibration member should be located below the test member (in a direction of the flow of body fluid along the second recess) so that the interaction between the body fluid and the test member can be detected from below without the calibration member disturbing this detection.

In an embodiment, the markers comprise a barcode and/or reference color fields. In this context, the term "barcode" may denote an optical machine-readable representation of data relating to the object to which it is attached. Such barcodes may systematically represent data by varying the widths and spacings of parallel lines (one-dimensional barcode). However, barcodes may also include rectangles, dots, hexagons and other geometric patterns in two dimensions (two-dimensional barcode). Reference colors can be used for color comparison and calibration of the detection system, particularly a camera. Calibration data written in the test unit can be read by the camera. A barcode can provide information about the type of a used test unit, a batch to which the test unit belongs, batch calibration information (i.e. lot to lot calibration resulting from the fact that different lots or batches may have different reactivity and shall therefore by calibrated differently) a date of expiry of the used test unit, etc.

In an embodiment, the detection unit is an electromagnetic radiation detection unit, particularly a digital camera, more particularly a CMOS (Complementary Metal Oxide Semiconductor) camera or a CCD (Charge-coupled Device) camera, configured for detecting electromagnetic radiation propagating from the test member and/or the calibration member to the detection unit. The measurement/detection of color development of the chemistry can be done by a digital camera (CCD, CMOS, etc.). Such cameras have become mass produced products which are cheap and have a high detection accuracy.

In an embodiment, the control device is configured as a portable device, particularly as a cellular phone. A cellular phone is particularly suitable as control device and shall be equipped with an adapter for receiving the test unit. Since cellular phones in many cases already contain cameras and light sources as well as processing resources, basically all necessary components for functioning as control device are already there.

The aspects defined above and further aspects of the invention are apparent from the examples of embodiment to be described hereinafter and are explained with reference to these examples of embodiment.

The invention will be described in more detail hereinafter with reference to examples of embodiment but to which the invention is not limited.
Fig. 1 is a schematic cross-section of a self-contained test unit for testing blood of a patient for determining the glucose level in the blood according to an exemplary embodiment of the invention.
Fig. 2 is a three-dimensional view of a single use test unit according to an exemplary embodiment of the invention.
Fig. 3 is a plan view of the test unit of Fig. 2.
Fig. 4 is a side view of a test unit of Fig. 2.
Fig. 5 is an explosive view of components of the test unit of Fig. 2.
Fig. 6A and Fig. 6B are different views of a part of a test unit according to an exemplary embodiment of the invention close to a position where transfer of blood from a patient to a capillary member of the test unit occurs.
Fig. 7 shows a part of the test unit of Fig. 6A and Fig. 6B when a blood drop enters the capillary member.
Fig. 8 shows two different views of the capillary member of a test unit according to an exemplary embodiment of the invention.
Fig. 9 and Fig. 10 show transfer of the blood from a bottom end of the capillary member towards a test member.
Fig. 10 is similar to Fig. 9 but additionally shows the position of the blood drop.
Fig. 11 is a three-dimensional view of a bottom part of a test unit according to an exemplary embodiment of the invention.
Fig. 12 is a top view of a test unit according to an exemplary embodiment of the invention.
Fig. 13 is a cross-sectional view of a test unit according to an exemplary embodiment of the invention.
Fig. 14 is a three-dimensional view of an integral void in a capillary member having a first recess with a circular cross section and a directly connected second recess with a triangular cross section.
Fig. 15 is a top view of two separate recesses of a capillary member according to an exemplary embodiment of the invention.
Fig. 16 is a top view of a circular first recess with a plurality of beak-shaped radially distributed second recesses according to an exemplary embodiment of the invention.
Fig. 17 is a top view of a circular first recess and a convex beak-shaped second recess.
Fig. 18 is a plan view of a circular first recess and a concave beak-shaped second recess according to an exemplary embodiment of the invention.
Fig. 19 is a plan view of a circular first recess with a directly connected rectangular strip-like or oblong rectangular second recess.
Fig. 20 is an integral void in a capillary member having a first basically circular recess with a connected smaller second circular recess.
Fig. 21 shows an arrangement of a self-contained test unit mounted on a cellular phone according to an exemplary embodiment of the invention.

The illustrations in the drawings are schematic. In different drawings, similar or identical elements are provided with the same reference signs.

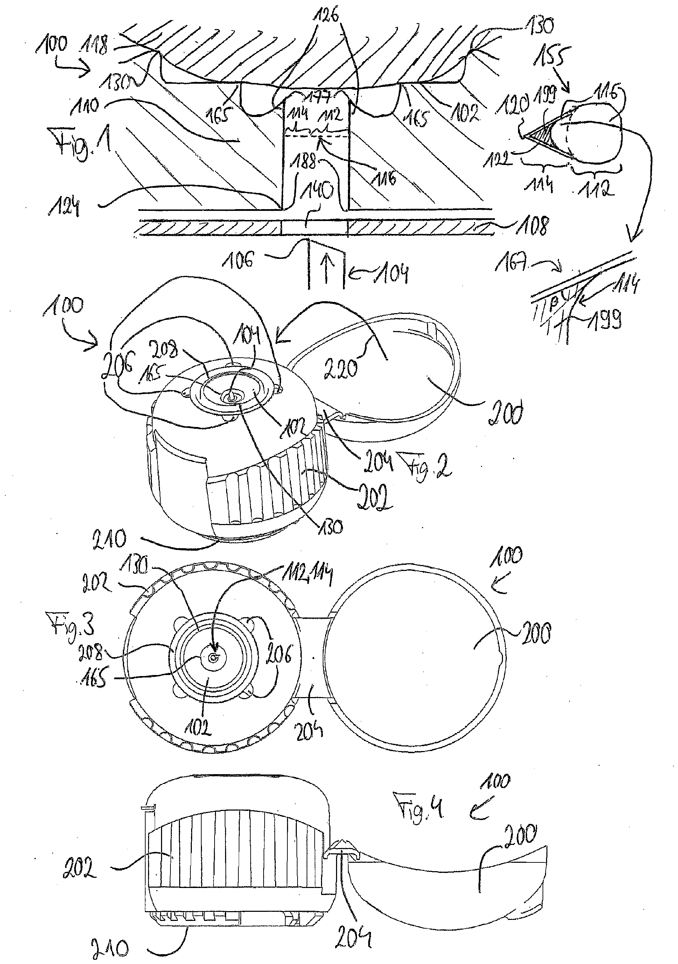

Fig. 1 is a cross-sectional view of a self-contained test unit 100 for testing blood of a diabetes patient for determining her or his glucose level according to an exemplary embodiment of the invention.

The test unit 100 comprises a fingertip receiving surface 102 for receiving a fingertip of a patient 118.

A metallic lancet 104 is movable along a direction indicated with an arrow in Fig. 1 and includes a sharp lancet tip 106. When the lancet tip 106 together with the lancet 104 moves in a vertical direction of Fig. 1, the tip 106 will pierce the skin of the fingertip to produce blood flow when the fingertip is received at the fingertip receiving surface 102 and when the lancet 104 has been moved so that the lancet tip 106 is in interaction with the patient's fingertip.

A test member 108 in the form of a transparent plastic ring is covered with a chemical configured for chemically reacting with glucose in the blood so as to characteristically change color. The degree of color change is indicative of the present glucose level of the patient 118. Thus, the test member 108 is capable of, upon interaction with the blood, supporting the determination of the glucose level in the blood. Fig. 1 furthermore shows that the test member 108 has a central hole 140 allowing the lancet 104 to move therethrough.

A capillary member 110 has a hollow first recess 112 configured for receiving and guiding the lancet 104. The capillary member 110 furthermore has a hollow second recess 114 which is configured for guiding the blood from the fingertip receiving surface 102 to the test member 108. Together, the first recess 112 and the second recess 114 form a continuous recess 116 within the capillary member 110. The capillary member 110 is preferably made of polycarbonate material and is formed as an injection moulded component.

A detail 155 in Fig. 1 shows the cross sectional shape of the integral recess 116. The detail 155 shows that the first recess 112, within which the lancet 104 exclusively and fully moves, has a circular shape. In contrast to this, the second recess 114, within which the basically circular cylindrical lancet 104 cannot move and along which the blood flows in a vertical direction of Fig. 1 has a basically triangular or beak shape. A dotted line within the integral recess 116 in detail 155 shows the functionally defined border between the first recess 112 and the second recess 114. Within an upper part of the vertical extension of the integral recess 116, the shape of the first recess 112 and the second recess 114 remains constant. However, at a lower end of the flow path, the second recess 114 may widen up and guides the blood or other body fluid laterally outwardly towards the test member 108.

The second recess 114 has a larger wall surface area to void volume ratio than the first recess 112. In other words, the surface area of the walls of the capillary member 110 delimiting the second recess 114 divided by the volume of the second recess 114 from an upper end 177 to a lower end 188 of the integral recess 116, is larger than the surface area of the walls of the capillary member 110 delimiting the first recess 112 divided by the volume of the first recess 112 from the upper end 177 to the lower end 188 of the integral recess 116. Therefore, the blood flowing from an upper end portion of the integral recess 116 to a lower end portion of the integral recess 116 will flow within an area 199 of the second recess 114, since the surface adhesion and the capillary forces for the aqueous body fluid are locally higher there as compared to the first recess 112. Within the second recess 114, aqueous body fluid is aligned directly to the capillary wall and has a concave surface at an end facing the first recess 112.

As can furthermore be taken from Fig. 1, the surface delimiting the beak-shaped triangular second recess 114 is locally covered with a coating 122 promoting wettability of this wall. The narrowest portion in the second recess 114 is close to an edge or corner 120 of the second recess 114. Coating 122 is configured so that, as can be seen in a detail 167 in Fig. 1, a contact angle β between a wall delimiting the second recess 114 and the body fluid partly wetting this wall is less than 90°. It is about 30° in the shown embodiment.

As can be furthermore taken from Fig. 1, the capillary member 110 has a sharp edge 124 at its end juxtaposing the test member 108. Therefore, the blood which flows downward along the second recess 114 will move along the sharp edge 124 and from there directly onto the chemical test member 108. In the shown embodiment, the sharp edge 124 has an L-shape, i.e. has a 90° angle.

The capillary member 110 has, at its upper end, a protrusion 126 juxtaposing the fingertip receiving surface 102. The first recess 112 as well as the second recess 114 extend through the protrusion 126. The geometrical conditions formed by the protrusion 126 force the blood from the fingertip into the integral recess 116.

The test unit 100 furthermore has a pressure cap 130 defined by the shape of the upper end of the test unit 110 and exerts pressure on the body part 118 to foster fluid flow out of a lanced side and into the second recess 114 of the capillary member 110. The pressure cap 130 surrounds the upper protrusion 126 of the capillary member 110. When a patient 118 presses her or his fingertip onto the pressure cap 130, the tissue will be locally compressed so that the lancet 104 pierces into a compressed, hard tissue which simplifies the process of blood extraction. Between protrusion 126 and pressure cup 130, a distance keeper 165 is provided which keeps the fingertip of the patient 118 spaced with regard to the protrusion 126 in order to prevent the wound from being unintentionally closed. The distance keeper 165 serves as an intermediate ring for distance control between finder and capillary inlet. The pressure cup serves as an outer ring for generating a pressure exerted on the skin of the finger that has the effect of promoting the flow of blood out of the lanced wound.

**Fig. 2, Fig. 3** and **Fig. 4** show different views of a test unit 100 according to an exemplary embodiment of the invention.

As can be taken from Fig. 2 showing a three-dimensional view of the test unit 100, the latter comprises a cap 200 which is integrally formed with a body member 202 and connected thereto with an integral hinge 204. The cap 200 is configured to be selectively opened for exposing the fingertip receiving surface 102 for use or closed for covering and shielding the fingertip receiving surface 102. A plurality of ventilation holes 206 are distributed circumferentially around the fingertip receiving part 102 of the test unit 100 and are configured for providing an air communication between the fingertip receiving surface 102 when being covered by the cap 200 and a part of the test unit 100 being exposed to atmospheric pressure of an environment. Therefore, upon closing the cap 200 by pivoting it along a curved arrow 220, no pressure pulses act on the components of the test unit 100 or on body fluid accommodated therein.

A support member 208 is provided in addition to the body member 202, and is movable with respect to the body member 202 between different positions and accommodates the lancet 104 (not shown).

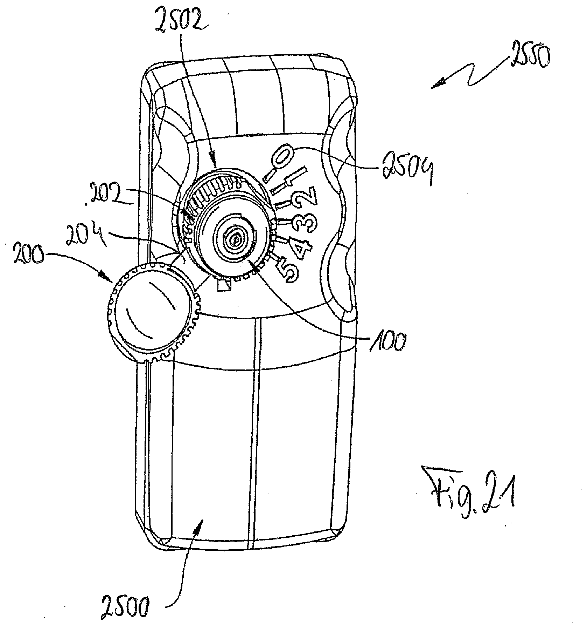

A control device attachment section 210 is configured for being attached to a test unit reception (see reference numeral 2502 in Fig. 21) of a control device (see reference numeral 2550 in Fig. 21).

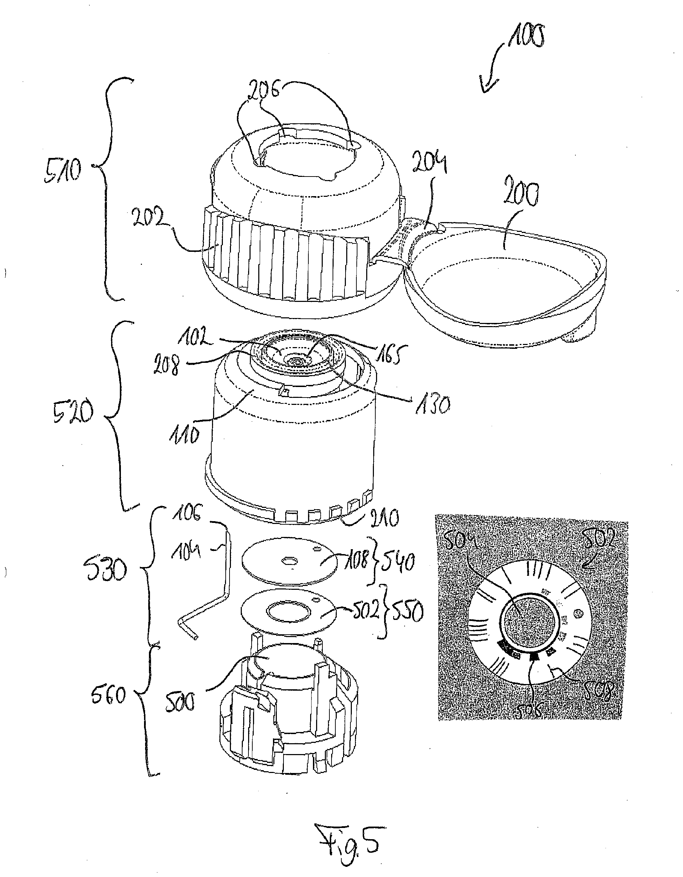

The test unit 100, which is shown in a three-dimensional view in Fig. 2, in a plan view in Fig. 3 and in a side view in Fig. 4, is shown in an exploded view in **Fig. 5****.**

Starting from the top and moving towards the bottom, Fig. 5 shows a cover part 510 designed for ergonomy and, lancing depth. The cover part 510 covers the capillary area before and after use.

A capillary part 520 including the capillary member 110 is shown below. It 520 has a defined capillary geometry, defines the lancing depth and guides a light guide 500 and the pressure cap 130.

Lancet part 530 shows the shape of the lancet 104. The lancet 104 shown with its tip 106 allows for the penetration of skin and after use, moves into a position where it can not be re-inserted into the skin. , so that the test unit 100 is not reusable for safety purposes. In other words, after the lancet 104 has been moved to pierce the body part of the patient 118, it will be retracted into an interior of the test unit 100 so that it cannot be moved out again.

A chemistry part 540 shows a chemistry plate as test member 108. It may comprise a reactive chemistry film on foil, and may have to be adapted to capillary action as well.

A calibration part 550 includes calibration plate 502 which carries information in form of calibration data, calibration colors, etc. It is shown in more detail on the right-hand side of Fig. 5. Calibration plate 502 is a ring having a central recess 504 being traversable by the lancet 104 and contains the information in form of markers 506, 508 which are arranged circumferentially and concentrically around the central recess 504. The markers include a barcode 508 and multiple reference color fields 506. Unlike the test member 108, the calibration member 502 is made from an opaque, for instance white, material on which the various color fields 506 and the barcode 508 are printed.

Furthermore, a support part 560 including support member 210 is shown in Fig. 5. The light guide 500 serves as an optical element for guiding light coming from the test member 108 or from the calibration plate 502 onto a CCD or CMOS camera of a control device. The support part 560 also serves for holding and accommodating the lancet 104.

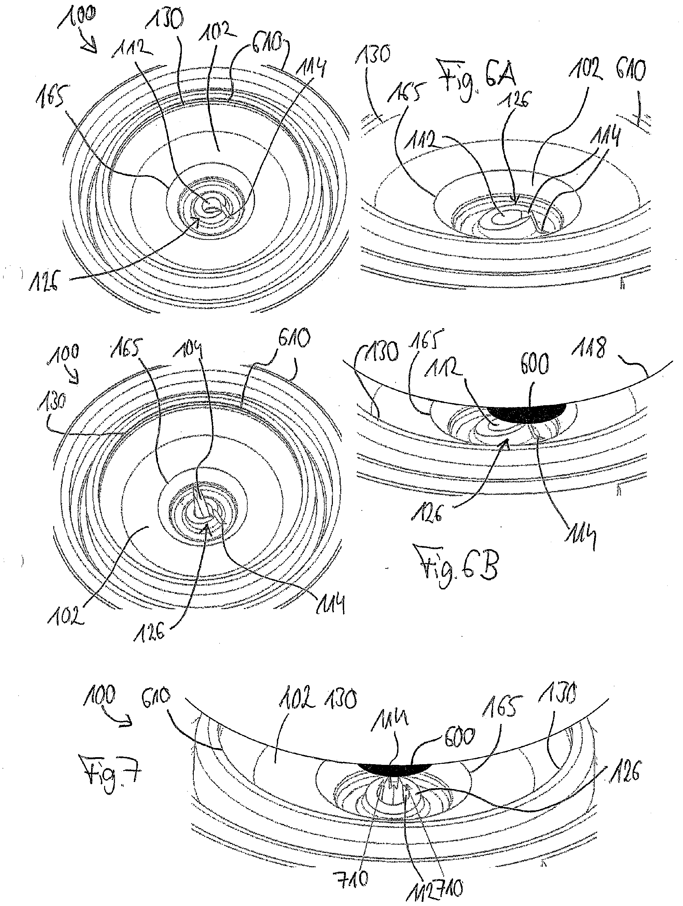

**Fig. 6A** and **Fig. 6B** illustrate a detail of the capillary entrance, i.e. the upper end of the capillary member 110 facing the fingertip of the patient 118. As can be taken from Fig. 6A and Fig. 6B, an upper surface of protrusion 126 is slanted relative to a plane defined by the top surface of one or more of circumferential rims 610 serving as pressure-generating rings of the pressure cup 130 of the body part receiving surface 102 in such a way that the second recess 114 extends through a portion of the protrusion 126 at an upper end of the slanted upper surface. The end of the second recess 114 is therefore closer to the fingertip than the end of the first recess 112.

Fig. 6B furthermore shows that a blood drop 600 then enters, as a consequence of the described geometry, the capillary via second recess 114 rather than via first recess 112. Thus, the capillary entrance has two extending areas (see reference numeral 710 in Fig. 7) which are narrowest close to the skin, which guarantees first contact to the blood drop 600 after lancing and leads the blood drop 600 to the transport capillary, more particularly to the narrower second recess 114 thereof.

**Fig. 7** is another view of this upper end portion of the capillary member 110 and illustrates the above described method of guiding the blood drop 600 specifically into the second recess 114.

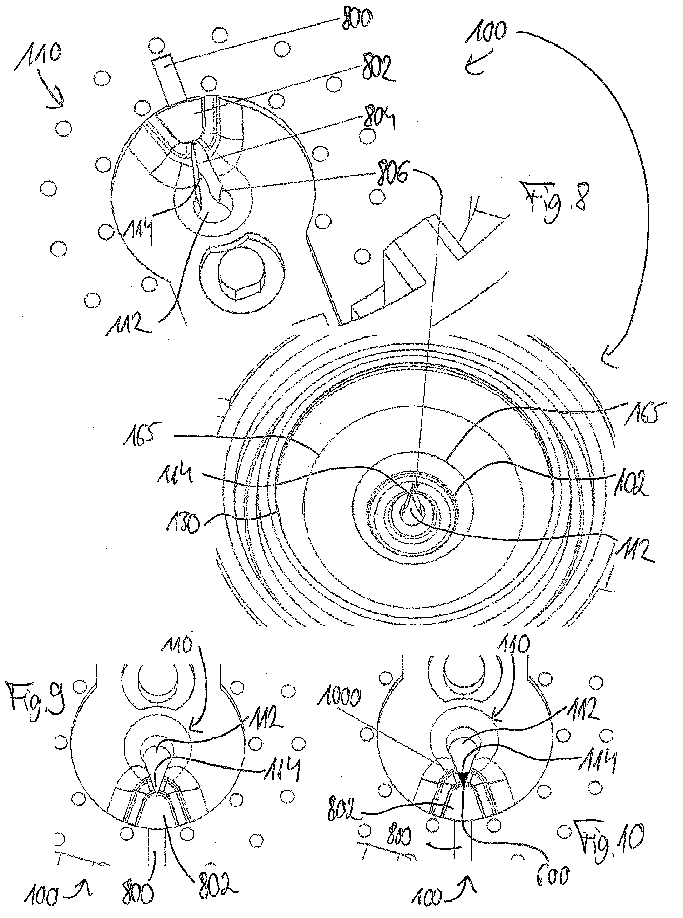

In **Fig. 8****,** a detailed view of a capillary design can be seen. This capillary design comprises a capillary entrance 806, a transport capillary 804 in fluid communication therewith, a reaction capillary 802 at which the reaction with the chemical on the test member 108 is initiated, and a connected suction capillary 800 into which the blood may be sucked.

Particularly, the transport capillary 804 channel is designed that the blood drop 600 is forced to flow in an edge area and not in the whole cross-section so that less blood volume is sufficient.

**Fig. 9** shows a further detailed view without a blood drop, and **Fig. 10** shows a corresponding view with blood drop 600. Fig. 10 shows that the blood drop 600 flows only in an edge, not in the whole cross-section. Thus, by using the effect of surface adhesion and capillary forces, the blood flow can be precisely controlled with simple measures. Reference numeral 1000 indicates that the blood drop 600 flows only in an edge, not in the whole cross-section of the channel.

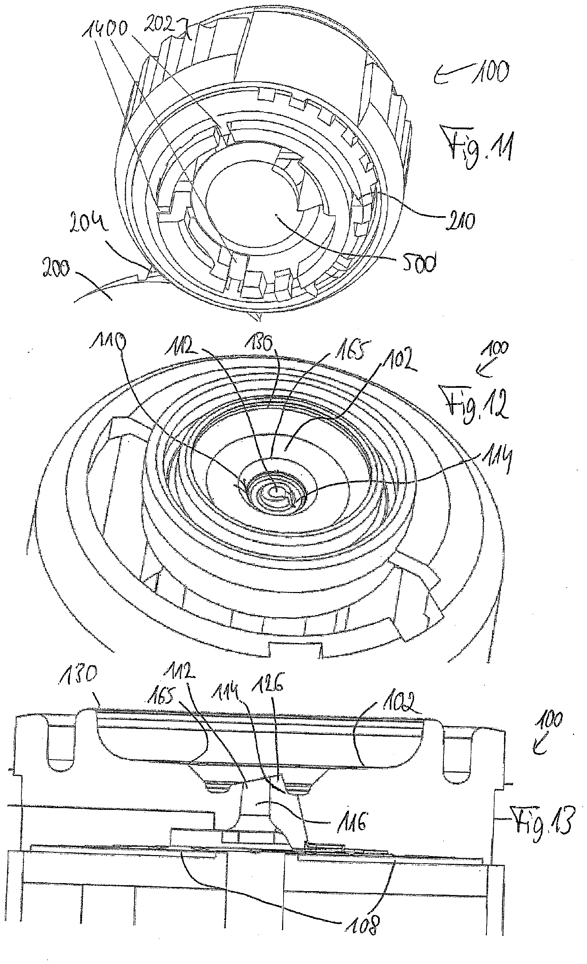

**Fig. 11** shows a three-dimensional view of a bottom of the test unit 100 and particularly shows guide rails 1400 for guiding support part 560 in capillary part 520.

**Fig. 12** shows a three-dimensional view of a top of the test unit 100 and **Fig. 13** shows a corresponding cross-sectional view.

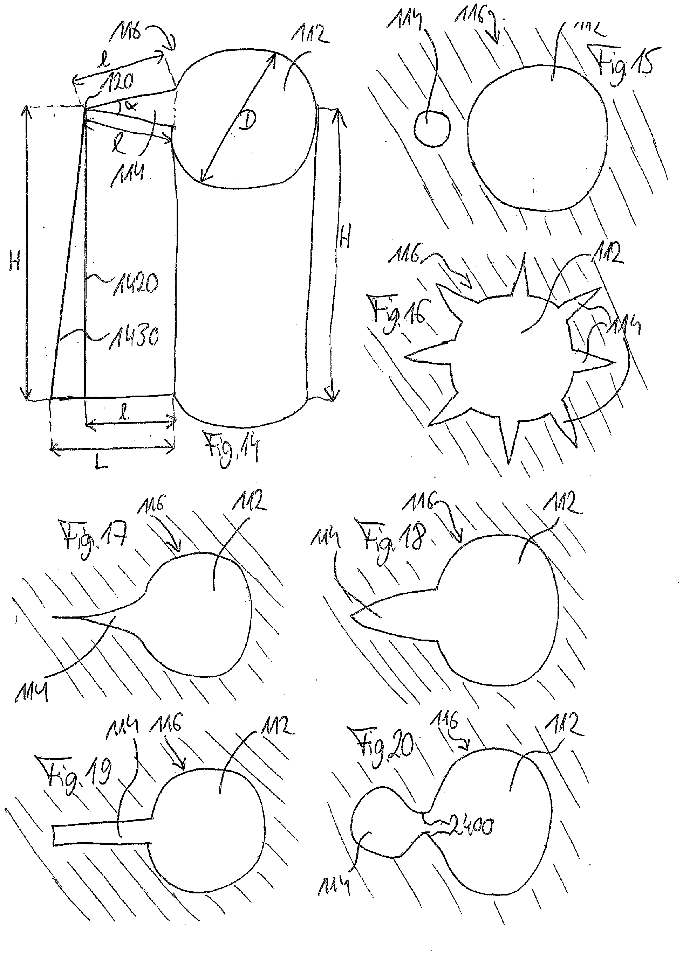

In **Fig. 14****,** a three-dimensional view of the first recess 112 and the second recess 114, together forming integral recess 116, is shown. Fig. 14 illustrates that the first recess 112 has a basically circular shape with a diameter D. The height (i.e. the length of the flow path of blood within the capillary member 110) of both the first recess 112 and the second recess 114 is denoted with H. Furthermore, the second recess 114 has a triangular shape with two side lengths I and has an angle α close to the tip or corner 120. If the dimensions D, H, I, α are selected so that the surface area to volume ratio of the second recess 114 is larger than the surface area to volume ratio of the first recess 112, the blood flow will take place predominantly within the second recess 114, more precisely close to the tip or corner 120 due to capillary effects.

In one alternative, the length I indicated at the body fluid inlet of the recess 116 of Fig. 14 remains constant along the entire (according to Fig. 14 vertical) extension of recess 116 so that the vertical extension of the corner end of the second recess 114 follows vertical line 1420. In another alternative, the length I indicated at the body fluid inlet of the recess 116 of Fig. 14 becomes larger towards the body fluid outlet (at the bottom of Fig. 14) and here assumes a value L>I so that the corner end of the second recess 114 follows slanted line 1430. The latter configuration is preferred since it directs the body fluids towards a lateral side of the second recess 114 thereby simplifying further transport of the body fluid from the bottom of the second recess 114 towards test member 108 (not shown in Fig. 14).

**Fig. 15** shows an alternative recess configuration according to another exemplary embodiment of the invention, in which both the first recess 112 and the second recess 114 are configured as circles, but are physically separated from one another so that no fluid communication is possible between them. Since the radius of the circle of the first recess 112 is significantly larger than the radius of the circle of the second recess 114, the blood flow will preferably take place along the path of second recess 114.

**Fig. 16** shows a first recess 112 in the shape of a circle to which a plurality of circumferentially distributed beaks 114 as second recess 114 are continuously connected. Again, the blood flow will take place along one or more of the beaks 114 in view of the strong capillary effects acting on the aqueous blood there.

**Fig. 17** shows another integral recess 116 having a circular first recess 112 and a directly connected convex second recess 114.

In **Fig. 18****,** a concave second recess 114 is directly connected to a circular first recess 112.

**Fig. 19** shows a circular first recess 112 with a strip-like second recess 114 having also a very small volume to surface ratio.

**Fig. 20** shows two connected basically circular recesses 112, 114 which are connected to one another by a curved interface section 2400. Again, blood flow will take place predominantly in second recess 114.

Fig. 14 to Fig. 20 illustrate that many recess designs are possible to achieve the effect of blood flow control due to locally differing capillary forces.

**Fig. 21** shows an arrangement 2550 for testing blood with regard to its glucose level and which can be used by diabetes patients. The arrangement 2550 comprises a self-contained test unit 100 as described above. Furthermore, the arrangement 2550 comprises a cellular phone 2500 having a test unit receiver2502 configured for detachably receiving and accommodating the test unit 100. Furthermore, the arrangement 2550 has a detection unit (not shown) in form of a CMOS camera or a CCD camera configured for detecting the glucose level by determining an impact of the interaction between the blood and a chemical test member 108 of the test unit 100.

In order to use the arrangement 2550, a user has simply to take a test unit 100 and mount it on the test unit receiver 2502 of the cellular phone 2550. The user may make an adjustment of a penetration depth of the lancet tip 106 on a scale 2504. After removing the cap 200 from the test unit 100 by pivoting so as to uncover the actual piercing surface of the test unit 100, the user may trigger motion of the lancet 104 out of the test unit 100 by pressing onto the uncovered part of the test unit 100. After that, the blood flows through the second recess 114 of the capillary towards the test member 108, wherein a detection of the measured blood with regard to the glucose level is then performed using the CCD camera and a processor of the cellular phone 2550.

It should be noted that the term "comprising" does not exclude other elements or steps and the "a" or "an" does not exclude a plurality. Also elements described in association with different embodiments may be combined.

It should also be noted that reference signs in the claims shall not be construed as limiting the scope of the claims.

Implementation of the invention is not limited to the preferred embodiments shown in the figures and described above. Instead, a multiplicity of variants are possible which use the solutions shown and the principle according to the invention even in the case of fundamentally different embodiments.

## Claims

1. A self contained test unit (100) for testing body fluid, the test unit (100) comprising :
a body part receiving surface (102) for receiving a patient's body part (118);
a movably mounted lancet (104) including a lancet tip (106) configured for piercing the skin of the patient to produce flow of body fluid when the patient's body part (118) is received at the body part receiving surface (102) and when the lancet (104) has been moved so that the lancet tip (106) is in interaction with the patient's body part (118);
a capillary member (110) having a first recess (112) configured for receiving the lancet (104) and having a second recess (114) configured for guiding the body fluid from the body part receiving surface (102) to a test member (108) for interacting with the body fluid to aid in the determination of information about body fluid components, wherein the second recess (114) has a larger surface area to volume ratio than the first recess (112), wherein the test member (108) is configured for interacting with the body fluid to aid in the determination of information about body fluid components, and the test member (108) has a central recess (504) being traversable by the lancet (104).

2. The test unit (100) of claim 1, wherein the first recess (112) and the second recess (114) are configured as an integral common recess (116), the second recess (114) being particularly a tapering extension extending from the first recess (112).

3. The test unit (100) of claim 1 or 2, wherein a cross-sectional area of the first recess (112), in a viewing direction perpendicular to the flow direction of the body fluid from the body part receiving surface (102) to the test member (108), is larger than a cross-sectional area of the second recess (114), in a viewing direction perpendicular to the flow direction of the body fluid from the body part receiving surface (102) to the test member (108).

4. The test unit (100) of any one of claims 1 to 3, wherein the second recess (114) has a surface area to volume ratio being at least twice, particularly at least five times, of a surface area to volume ratio of the first recess (112).

5. The test unit (100) of any one of claims 1 to 4, wherein the first recess (112) has a generally circular shape in a viewing direction generally
perpendicular to the flow direction of the body fluid from the body part receiving surface (102) to the test member (108).

6. The test unit (100) of any one of claims 1 to 5, wherein the second recess (114) is a generally triangular structure, particularly has the shape of an isosceles triangle, in a viewing direction perpendicular to the flow direction of the body fluid from the body part receiving surface (102) to the test member (108), particularly is a V-shaped notch.

7. The test unit (100) of claim 6, wherein the triangular structure (114) has an angle (a) at an end opposing the first recess (112) in a range between 20° and 120°, particularly in a range between 30° and 70°.

8. The test unit (100) of claim 6 or 7, wherein the triangular structure (114) has side lengths (I), particularly two equal side lengths (I), extending from the first recess (112) towards a tip (120) in a range between 0.01 mm and 2 mm, particularly in a range between 0.1 mm and 1 mm.

9. The test unit (100) of any one of claims 1 to 8, wherein at least the capillary member (110) is made of a plastic material, particularly of one of the group consisting of polycarbonate, polystyrene, polybutylene terephthalate, polyethylene terephthalate, poly(methyl methacrylate, polyvinyl chloride, and polypropylene.

10. The test unit (100) of any one of claims 1 to 9, wherein at least walls of the capillary member (110) delimiting the second recess (114), particularly walls of the capillary member (110) delimiting the first recess (112) and the second recess (114), are surface-configured to provide a contact angle (β) of less than 90°, particularly of less than 60°, between a wall delimiting the second recess (114) and the body fluid partly wetting this wall, particularly are coated with a hydrophilic coating (122), are chemically surface activated, or have a physical surface structure.

11. The test unit (100) of any one of claims 1 to 10, wherein the cross-sectional shape of the second recess (114), in a viewing direction generally perpendicular to the flow direction of the body fluid, widens up, particularly continuously widens up, along a flow path of the body fluid along the second recess (114).

12. The test unit (100) of any one of claims 1 to 11, wherein at least the capillary member (110) is an injection moulded component.

13. The test unit (100) of any one of claims 1 to 12, wherein the capillary member (110) has a sharp edge (124) at its end juxtaposing the test member (108).

14. The test unit (100) of claim 13, wherein the sharp edge (124) has one of the group consisting of a V-shape, an L-shape, and a curvature with a radius of less than 0.1 mm, particularly of less than 0.05 mm.

15. The test unit (100) of any one of claims 1 to 14, wherein the capillary member (110) has a protrusion (126) at its end juxtaposing the body part receiving surface (102), wherein the first recess (112) and the second recess (114) extend through the protrusion (126).

16. The test unit (100) of claim 15, wherein the first recess (112) extends through a central portion of the protrusion (126) and the second recess (114) extends through a lateral portion of the protrusion (126).

17. The test unit (100) of claim 15 or 16, wherein an upper surface of the protrusion (126) is slanted in such a way that the second recess (114) extends through a portion of the protrusion (126) at an upper end of the slanted upper surface.

18. The test unit (100) of any of claims 15 to 17, wherein an upper surface of the protrusion (126) has two tips between which the second recess (114) extends through the protrusion (126).

19. The test unit (100) of any one of claims 1 to 18, comprising a cap (200), particularly integrally formed with a body member (202) of the test unit (100), more particularly pivotably connected to a body member (202) of the test unit (100) by an integral hinge (204), the cap (200) being configured to be selectively opened for exposing the body part receiving surface (102) or closed for covering the body part receiving surface (102).

20. The test unit (100) of claim 19, comprising at least one ventilation hole (206) for providing an air communication between the body part receiving surface (102) to be covered by the cap (200) and another part of the test unit (100) being permanently exposed to atmospheric pressure.

21. The test unit (100) of any one of claims 1 to 20, comprising a body member (202) and a support member (208) moveable with respect to the body member (202) between different positions, the support member (208) accommodating the lancet (104).

22. The test unit (100) of claim 21, wherein the support member (208) comprises a control device attachment section (210) configured for being attached to a test unit reception (2502) of a control device (2500).

23. The test unit (100) of any one of claims 1 to 22, comprising a pressure cup (130) for exerting pressure on the body part (118) to foster fluid flow out of a lanced site and into the second recess (114) of the capillary member (110).

24. The test unit (100) of claim 23, wherein the pressure cup (130) comprises a circumferential rim which surrounds an upper end, particularly an upper protrusion (126), of the capillary member (110).

25. The test unit (100) of claims 21 and 23, wherein the test unit (100) is configured so that exerting pressure on the pressure cup (130) simultaneously moves the support member (208) with respect to the body member (202) to thereby move the lancet (104) in such a way that the lancet tip (106) pierces the skin of the body part (118) upon exerting pressure on the pressure cup (130).

26. The test unit (100) of claim 25, wherein the test unit (100) is configured so that the lancet (104) is further moved to be accommodated in an inactive state within an interior of the test unit (100) upon continued exertion of pressure after the piercing.

27. The test unit (100) of any one of claims 1 to 26, configured as a self contained disposable test unit (100) which can only be used once.

28. The test unit (100) of claim 1, wherein the test member (108) is a transparent ring which comprises a substance which is configured for chemically reacting with the body fluid.

29. The test unit (100) of claim 28, wherein the test member (108) is configured for chemically reacting with the body fluid to thereby create a reaction product being detectable, particularly quantitatively detectable, photometrically.

30. The test unit (100) of claim 29, comprising an optical member (500), particularly a light guide, being configured for optically manipulating, particularly for guiding, electromagnetic radiation from at least one of the test member (108) and a calibration member (502) towards a detection unit of a control device (2500) on which the test unit (100) is received.

31. The test unit (100) of any one of claims 1 to 30, comprising a calibration member (502) configured for containing detectable information for calibration of the test unit (100).

32. The test unit (100) of claim 31, wherein the calibration member (502) is a ring having a central recess (504) being traversable by the lancet (104) and containing the information in the form of markers (506, 508) being arranged circumferentially around the central recess (504).

33. The test unit (100) of claim 32, wherein the markers comprise at least one of the group consisting of a barcode (508) and at least one reference color field (506).

34. The test unit (100) of any one of claims 1 to 33, wherein a volume of the second recess (114) is less than 400 nl, particularly less than 200 nl .

35. An apparatus (2550) for testing body fluid, the arrangement (2550) comprising :
a self contained test unit (100) according to any of claims 1 to 34;
a control device (2500) having :
a test unit reception (2502) configured for detachably receiving the test unit (100);
a detection unit configured for detecting the information about body fluid components by determining an impact of the interaction between the body fluid and the test member (108).

36. The apparatus (2550) of claim 35, wherein the detection unit is an electromagnetic radiation detection unit, particularly a digital camera, more particularly a CMOS camera or a CCD camera, configured for detecting electromagnetic radiation propagating from the test member (108) to the detection unit.

37. The apparatus (2550) of claim 35 or 36, wherein the control device (2500) is configured as a portable device, for example as a cellular phone.

38. A method of testing body fluid using a self contained test unit (100), the method comprising :
receiving a patient's body part (118) on a body part receiving surface (102) of the test unit (100);
moving a lancet (104), having a lancet tip (106), of the test unit (100) through a first recess (112) of a capillary member (110) of the test unit (100) so that the lancet tip (106) is in interaction with the patient's body part (118) for piercing the skin of the patient to produce flow of body fluid;
bringing a test member (108) of the test unit (100) in interaction with the body fluid by guiding the body fluid from the body part receiving surface (102) through a second recess (114) of the capillary member (110) to the test member (108) to aid in the determination of information about body fluid components;
wherein the second recess (114) has a larger surface area to volume ratio than the first recess (112), wherein the test member (108) is configured for interacting with the body fluid to aid in the determination of information about body fluid components, and the test member (108) has a central recess (504) being traversable by the lancet (104).

## Patentansprüche

1. Eine in sich geschlossene Testeinheit (100) zum Testen von Körperflüssigkeit, wobei die Testeinheit (100) folgende Merkmale aufweist:
eine Körperteilaufnahmeoberfläche (102) zum Aufnehmen eines Körperteils eines Patienten (118);
eine bewegbar angebrachte Lanzette (104), die eine Lanzettenspitze (106) umfasst, die zum Durchstechen der Haut des Patienten konfiguriert ist, um einen Fluss von Körperflüssigkeit zu erzeugen, wenn der Körperteil des Patienten (118) an der Körperteilaufnahmeoberfläche (102) aufgenommen wird und wenn die Lanzette (104) so bewegt wurde, dass sich die Lanzettenspitze (106) in Wechselwirkung mit dem Körperteil des Patienten (118) befindet;
ein Kapillarbauglied (110), das eine erste Aussparung (112), die zum Aufnehmen der Lanzette (104) konfiguriert ist, und eine zweite Aussparung (114) aufweist, die dazu konfiguriert ist, die Körperflüssigkeit von der Körperteilaufnahmeoberfläche (102) zu einem Testbauglied (108) zu führen, um mit der Körperflüssigkeit in Wechselwirkung zu stehen, um bei der Bestimmung von Informationen über Körperflüssigkeitskomponenten behilflich zu sein, wobei die zweite Aussparung (114) ein größeres Oberfläche/Volumen-Verhältnis aufweist als die erste Aussparung (112), wobei das Testbauglied (108) dazu konfiguriert ist, mit der Körperflüssigkeit in Wechselwirkung zu stehen, um bei der Bestimmung von Informationen über Körperflüssigkeitskomponenten behilflich zu sein, und das Testbauglied (108) eine zentrale Aussparung (504) aufweist, die seitens der Lanzette (104) durchquert werden kann.

2. Die Testeinheit (100) gemäß Anspruch 1, bei der die erste Aussparung (112) und die zweite Aussparung (114) als in einem Stück vorliegende gemeinsame Aussparung (116) konfiguriert sind, wobei die zweite Aussparung (114) insbesondere eine sich verjüngende Ausdehnung ist, die sich von der ersten Aussparung (112) aus erstreckt.

3. Die Testeinheit (100) gemäß Anspruch 1 oder 2, bei der eine Querschnittsfläche der ersten Aussparung (112) in einer Betrachtungsrichtung, die senkrecht zu der Fließrichtung der Körperflüssigkeit von der Körperteilaufnahmeoberfläche (102) zu dem Testbauglied (108) ist, größer ist als eine Querschnittsfläche der zweiten Aussparung (114) in einer Betrachtungsrichtung, die senkrecht zu der Fließrichtung der Körperflüssigkeit von der Körperteilaufnahmeoberfläche (102) zu dem Testbauglied (108) ist.

4. Die Testeinheit (100) gemäß einem der Ansprüche 1 bis 3, bei der die zweite Aussparung (114) ein Oberfläche/Volumen-Verhältnis aufweist, das zumindest das Doppelte, insbesondere zumindest das Fünffache, eines Oberfläche/Volumen-Verhältnisses der ersten Aussparung (112) beträgt.

5. Die Testeinheit (100) gemäß einem der Ansprüche 1 bis 4, bei der die erste Aussparung (112) in einer Betrachtungsrichtung, die allgemein senkrecht zu der Fließrichtung der Körperflüssigkeit von der Körperteilaufnahmeoberfläche (102) zu dem Testbauglied (108) ist, eine allgemein kreisförmige Gestalt aufweist.

6. Die Testeinheit (100) gemäß einem der Ansprüche 1 bis 5, bei der die zweite Aussparung (114) eine allgemein dreieckige Struktur ist, insbesondere die Form eines gleichschenkligen Dreiecks aufweist, in einer Betrachtungsrichtung, die senkrecht zu der Fließrichtung der Körperflüssigkeit von der Körperteilaufnahmeoberfläche (102) zu dem Testbauglied (108) ist, insbesondere eine V-förmige Einkerbung ist.

7. Die Testeinheit (100) gemäß Anspruch 6, bei der die dreieckige Struktur (114) an einem der ersten Aussparung (112) gegenüberliegenden Ende einen Winkel (a) in einem Bereich zwischen 20° und 120°, insbesondere in einem Bereich zwischen 30° und 70°, aufweist.

8. Die Testeinheit (100) gemäß Anspruch 6 oder 7, bei der die dreieckige Struktur (114) Seitenlängen (I), insbesondere zwei gleiche Seitenlängen (I), die sich von der ersten Aussparung (112) hin zu einer Spitze (120) erstrecken, in einem Bereich zwischen 0,01 mm und 2 mm, insbesondere in einem Bereich zwischen 0,1 mm und 1 mm, aufweist.

9. Die Testeinheit (100) gemäß einem der Ansprüche 1 bis 8, bei der zumindest das Kapillarbauglied (110) aus einem Kunststoffmaterial hergestellt ist, insbesondere aus einem der Gruppe, die aus Polykarbonat, Polystyrol, Polybutylenterephthalat, Polyethylenterephthalat, Poly(Methylmethacrylat, Polyvinylchlorid und Polypropylen besteht.

10. Die Testeinheit (100) gemäß einem der Ansprüche 1 bis 9, bei der zumindest Wände des Kapillarbauglieds (110), die die zweite Aussparung (114) begrenzen, insbesondere Wände des Kapillarbauglieds (110), die die erste Aussparung (112) und die zweite Aussparung (114) begrenzen, dahin gehend oberflächenkonfiguriert sind, einen Kontaktwinkel (ß) von weniger als 90°, insbesondere von weniger als 60°, zwischen einer die zweite Aussparung (114) begrenzenden Wand und der diese Wand teilweise benetzenden Körperflüssigkeit bereitzustellen, insbesondere mit einer hydrophilen Beschichtung (122) beschichtet sind, chemisch oberflächenaktiviert sind oder eine physische Oberflächenstruktur aufweisen.

11. Die Testeinheit (100) gemäß einem der Ansprüche 1 bis 10, bei der sich die Querschnittsform der zweiten Aussparung (114) in einer Betrachtungsrichtung, die allgemein senkrecht zu der Fließrichtung der Körperflüssigkeit von der Körperteilaufnahmeoberfläche (102) zu dem Testbauglied (108) ist, entlang eines Strömungswegs der Körperflüssigkeit entlang der zweiten Aussparung (114) ausweitet, insbesondere kontinuierlich ausweitet.

12. Die Testeinheit (100) gemäß einem der Ansprüche 1 bis 11, bei der zumindest das Kapillarbauglied (110) eine spritzgegossene Komponente ist.

13. Die Testeinheit (100) gemäß einem der Ansprüche 1 bis 12, bei der das Kapillarbauglied (110) an seinem an das Testbauglied (108) anschließende Ende eine scharfe Kante (124) aufweist.

14. Die Testeinheit (100) gemäß Anspruch 13, bei der die scharfe Kante (124) eine aus der Gruppe aufweist, die aus einer V-Form, einer L-Form und einer Krümmung mit einem Radius von weniger als 0,1 mm, insbesondere von weniger als 0,05 mm, besteht.

15. Die Testeinheit (100) gemäß einem der Ansprüche 1 bis 14, bei der das Kapillarbauglied (110) an seinem Ende, das an die Körperteilaufnahmeoberfläche (102) anschließt, einen Vorsprung (126) aufweist, wobei sich die erste Aussparung (112) und die zweite Aussparung (114) durch den Vorsprung (126) hindurch erstrecken.

16. Die Testeinheit (100) gemäß Anspruch 15, bei der sich die erste Aussparung (112) durch einen mittleren Abschnitt des Vorsprungs (126) hindurch erstreckt und sich die zweite Aussparung (114) durch einen lateralen Abschnitt des Vorsprungs (126) hindurch erstreckt.

17. Die Testeinheit (100) gemäß Anspruch 15 oder 16, bei der eine obere Oberfläche des Vorsprungs (126) derart schräg ist, dass sich die zweite Aussparung (114) an einem oberen Ende der schrägen oberen Oberfläche durch einen Abschnitt des Vorsprungs (126) hindurch erstreckt.

18. Die Testeinheit (100) gemäß Anspruch 15 bis 17, bei der eine obere Oberfläche des Vorsprungs (126) zwei Spitzen aufweist, zwischen denen sich die zweite Aussparung (114) durch den Vorsprung (126) hindurch erstreckt.

19. Die Testeinheit (100) gemäß einem der Ansprüche 1 bis 18, die eine Kappe (200) aufweist, die insbesondere in einem Stück mit einem Körperbauglied (202) der Testeinheit (100) gebildet ist, die genauer gesagt mittels eines Filmgelenks (204) schwenkbar mit einem Körperbauglied (202) der Testeinheit (100) verbunden ist, wobei die Kappe (200) dazu konfiguriert ist, selektiv geöffnet zu werden, um die Körperteilaufnahmeoberfläche (102) freizulegen, oder geschlossen zu werden, um die Körperteilaufnahmeoberfläche (102) abzudecken.

20. Die Testeinheit (100) gemäß Anspruch 19, die zumindest ein Lüftungsloch (206) zum Bereitstellen einer Luftkommunikation zwischen der Körperteilaufnahmeoberfläche (102), die durch die Kappe (200) abgedeckt werden soll, und einem anderen Teil der Testeinheit (100), der dauerhaft atmosphärischem Druck ausgesetzt ist, aufweist.

21. Die Testeinheit (100) gemäß einem der Ansprüche 1 bis 20, die ein Körperbauglied (202) und ein Tragebauglied (208), das bezüglich des Körperbauglieds (202) zwischen verschiedenen Positionen bewegbar ist, aufweist, wobei das Tragebauglied (208) die Lanzette (104) beherbergt.

22. Die Testeinheit (100) gemäß Anspruch 21, bei der das Tragebauglied (208) ein Steuervorrichtungsbefestigungssegment (210) aufweist, das dazu konfiguriert ist, an einer Testeinheitsaufnahme (2502) einer Steuervorrichtung (2500) befestigt zu werden.

23. Die Testeinheit (100) gemäß einem der Ansprüche 1 bis 22, die einen Druckbecher (130) zum Ausüben eines Drucks auf den Körperteil (118) aufweist, um einen Flüssigkeitsstrom aus einer mit einer Lanzette aufgestochenen Stelle heraus und in die zweite Aussparung (114) des Kapillarbauglieds (110) zu befördern.

24. Die Testeinheit (100) gemäß Anspruch 23, bei der der Druckbecher (130) einen Umfangsrand aufweist, der ein oberes Ende, insbesondere einen oberen Vorsprung (126), des Kapillarbauglieds (110) umgibt.

25. Die Testeinheit (100) gemäß den Ansprüchen 21 und 23, wobei die Testeinheit (100) so konfiguriert ist, dass ein Ausüben eines Drucks auf den Druckbecher (130) gleichzeitig das Tragebauglied (208) bezüglich des Körperbauglieds (202) bewegt, um dadurch die Lanzette (104) derartig zu bewegen, dass die Lanzettenspitze (106) auf das Ausüben eines Drucks auf den Druckbecher (130) hin die Haut des Körperteils (118) durchsticht.

26. Die Testeinheit (100) gemäß Anspruch 25, wobei die Testeinheit (100) so konfiguriert ist, dass die Lanzette (104) weiter bewegt wird, um auf ein fortgesetztes Ausüben von Druck nach dem Durchstechen hin in einem inaktiven Zustand in einem Inneren der Testeinheit (100) untergebracht zu werden.

27. Die Testeinheit (100) gemäß einem der Ansprüche 1 bis 26, die als in sich geschlossene Einweg-Testeinheit (100) konfiguriert ist, die nur einmal verwendet werden kann.

28. Die Testeinheit (100) gemäß Anspruch 1, bei der das Testbauglied (108) ein transparenter Ring ist, der eine Substanz aufweist, die für eine chemische Reaktion mit der Körperflüssigkeit konfiguriert ist.

29. Die Testeinheit (100) gemäß Anspruch 28, bei der das Testbauglied (108) für eine chemische Reaktion mit der Körperflüssigkeit konfiguriert ist, um dadurch ein Reaktionsprodukt zu erzeugen, das photometrisch erfassbar, insbesondere quantitativ erfassbar, ist

30. Die Testeinheit (100) gemäß Anspruch 29, die ein optisches Bauglied (500), insbesondere einen Lichtleiter, aufweist, dsd dazu konfiguriert ist, elektromagnetische Strahlung von zumindest entweder dem Testbauglied (108) und/oder einem Kalibrierungsbauglied (502) optisch zu manipulieren, insbesondere hin zu einer Erfassungseinheit einer Steuervorrichtung (2500), an der die Testeinheit (100) aufgenommen wird, zu lenken.

31. Die Testeinheit (100) gemäß einem der Ansprüche 1 bis 30, die ein Kalibrierungsbauglied (502) aufweist, das dazu konfiguriert ist, erfassbare Informationen zur Kalibrierung der Testeinheit (100) zu enthalten.

32. Die Testeinheit (100) gemäß Anspruch 31, bei der das Kalibrierungsbauglied (502) ein Ring ist, der eine mittlere Aussparung (504) aufweist, die seitens der Lanzette (104) durchquert werden kann und die die Informationen in Form von Markierungen (506, 508) enthält, die umfangsmäßig um die mittlere Aussparung (504) herum angeordnet sind.

33. Die Testeinheit (100) gemäß Anspruch 32, bei der die Markierungen zumindest eines aus der Gruppe aufweisen, die aus einem Strichcode (508) und zumindest einem Referenzfarbfeld (506) besteht.

34. Die Testeinheit (100) gemäß einem der Ansprüche 1 bis 33, bei der ein Volumen der zweiten Aussparung (114) weniger als 400 nl, insbesondere weniger als 200 nl, beträgt.

35. Eine Vorrichtung (2550) zum Testen einer Körperflüssigkeit, wobei die Anordnung (2550) folgende Merkmale aufweist:
eine in sich geschlossene Testeinheit (100) gemäß einem der Ansprüche 1 bis 34;
eine Steuervorrichtung (2500), die folgende Merkmale aufweist:
eine Testeinheitsaufnahme (2502), die dazu konfiguriert ist, die Testeinheit (100) auf lösbare Weise aufzunehmen;
eine Erfassungseinheit, die zum Erfassen der Informationen über Körperflüssigkeitskomponenten durch Bestimmen einer Auswirkung der Wechselwirkung zwischen der Körperflüssigkeit und dem Testbauglied (108) konfiguriert ist.

36. Die Vorrichtung (2550) gemäß Anspruch 35, bei der die Erfassungseinheit eine Einheit zur Erfassung elektromagnetischer Strahlung ist, insbesondere eine Digitalkamera, genauer gesagt eine CMOS-Kamera oder eine CCD-Kamera, die dazu konfiguriert ist, elektromagnetische Strahlung zu erfassen, die sich von dem Testbauglied (108) zu der Erfassungseinheit hin ausbreitet.

37. Die Vorrichtung (2550) gemäß Anspruch 35 oder 36, bei der die Steuervorrichtung (2500) als tragbare Vorrichtung, beispielsweise als Mobiltelefon, konfiguriert ist.

38. Ein Verfahren zum Testen einer Körperflüssigkeit unter Verwendung einer in sich geschlossenen Testeinheit (100), wobei das Verfahren folgende Schritte aufweist:
Aufnehmen eines Körperteils eines Patienten (118) auf einer Körperteilaufnahmeoberfläche (102) der Testeinheit (100);
Bewegen einer Lanzette (104), die eine Lanzettenspitze (106) aufweist, der Testeinheit (100) durch eine erste Aussparung (112) eines Kapillarbauglieds (110) der Testeinheit (100) hindurch, so dass die Lanzettenspitze (106) in Wechselwirkung mit dem Körperteil des Patienten (118) steht, um die Haut des Patienten zu durchstechen, um einen Fluss von Körperflüssigkeit zu erzeugen;
In-Wechselwirkung-Bringen eines Testbauglieds (108) der Testeinheit (100) mit der Körperflüssigkeit durch Führen der Körperflüssigkeit von der Körperteilaufnahmeoberfläche (102) durch eine zweite Aussparung (114) des Kapillarbauglieds (110) hindurch zu dem Testbauglied (108), um bei der Bestimmung von Informationen über Körperflüssigkeitskomponenten behilflich zu sein;
wobei die zweite Aussparung (114) ein größeres Oberfläche/Volumen-Verhältnis aufweist als die erste Aussparung (112), wobei das Testbauglied (108) dazu konfiguriert ist, mit der Körperflüssigkeit in Wechselwirkung zu stehen, um bei der Bestimmung von Informationen über Körperflüssigkeitskomponenten behilflich zu sein, und das Testbauglied (108) eine zentrale Aussparung (504) aufweist, die seitens der Lanzette (104) durchquert werden kann.

## Revendications

1. Unité de test autonome (100) pour tester un fluide corporel, l'unité de test (100) comprenant:
une surface de réception de partie de corps (102) destinée à recevoir une partie de corps d'un patient (118);
une lancette (104) montée de manière mobile comportant une pointe de lancette (106) configurée pour percer la peau du patient pour produire un écoulement de fluide corporel lorsque la partie de corps du patient (118) est reçue sur la surface de réception de partie de corps (102) et lorsque la lancette (104) a été déplacée de sorte que la pointe de lancette (106) soit en interaction avec la partie de corps du patient (118);
un élément capillaire (110) présentant un premier évidement (112) configuré pour recevoir la lancette (104) et présentant un deuxième évidement (114) configuré pour guider le fluide corporel de la surface de réception de partie de corps (102) vers un élément de test (108) pour interagir avec le fluide corporel pour aider à déterminer les informations sur les composants de fluide corporel, où le deuxième évidement (114) présente un rapport superficie/volume plus grand que le premier évidement (112), où l'élément de test (108) est configuré pour interagir avec le fluide corporel pour aider à déterminer les informations sur les composants de fluide corporel, et l'élément de test (108) présente un évidement central (504) pouvant être traversé par la lancette (104).

2. Unité de test (100) selon la revendication 1, dans laquelle le premier évidement (112) et le deuxième évidement (114) sont configurés comme un évidement commun intégral (116), le deuxième évidement (114) étant en particulier un prolongement conique s'étendant à partir du premier évidement (112).

3. Unité de test (100) selon la revendication 1 ou 2, dans laquelle une superficie de section du premier évidement (112), dans une direction de vue perpendiculaire à la direction d'écoulement du fluide corporel de la surface de réception de partie du corps (102) vers l'élément de test (108), est plus grande qu'une superficie de section du deuxième évidement (114), dans une direction de vue perpendiculaire à la direction d'écoulement du fluide corporel de la surface de réception de partie de corps (102) vers l'élément de test (108).

4. Unité de test (100) selon l'une quelconque des revendications 1 à 3, dans laquelle le deuxième évidement (114) présente un rapport superficie/volume qui est au moins deux fois, en particulier au moins cinq fois, un rapport superficie-volume du premier évidement (112).

5. Unité de test (100) selon l'une quelconque des revendications 1 à 4, dans laquelle le premier évidement (112) présente une forme généralement circulaire dans une direction de vue généralement perpendiculaire à la direction d'écoulement du fluide corporel de la surface de réception de partie de corps (102) vers l'élément de test (108).

6. Unité de test (100) selon l'une quelconque des revendications 1 à 5, dans laquelle le deuxième évidement (114) est une structure généralement triangulaire, présente en particulier la forme d'un triangle isocèle, dans une direction de vue perpendiculaire à la direction d'écoulement du fluide corporel de la surface de réception de partie de corps (102) vers l'élément de test (108), est en particulier une encoche en forme de V.

7. Unité de test (100) selon la revendication 6, dans laquelle la structure triangulaire (114) présente un angle (a) à une extrémité opposée au premier évidement (112) dans une plage comprise entre 20° et 120°, en particulier dans une plage comprise entre 30° et 70°.

8. Unité de test (100) selon la revendication 6 ou 7, dans laquelle la structure triangulaire (114) présente des longueurs latérales (I), en particulier deux longueurs latérales égales (I), s'étendant du premier évidement (112) vers une pointe (120) dans une plage comprise entre 0,01 mm et 2 mm, en particulier dans une plage comprise entre 0,1 mm et 1 mm.

9. Unité de test (100) selon l'une quelconque des revendications 1 à 8, dans laquelle au moins l'élément capillaire (110) est réalisé en une matière plastique, en particulier l'une du groupe constitué de polycarbonate, de polystyrène, de téréphtalate de polybutylène, de téréphtalate de polyéthylène, de (poly)méthacrylate de méthyle, de chlorure de polyvinyle, et de polypropylène.

10. Unité de test (100) selon l'une quelconque des revendications 1 à 9, dans laquelle au moins les parois de l'élément capillaire (110) délimitant le deuxième évidement (114), en particulier les parois de l'élément capillaire (110) délimitant le premier évidement (112) et le deuxième évidement (114) sont configurées en surface pour créer un angle de contact (β) de moins de 90°, en particulier de moins de 60°, entre une paroi délimitant le deuxième évidement (114) et le fluide corporel mouillant partiellement cette paroi, en particulier sont revêtues d'un revêtement hydrophile (122), sont chimiquement activés en surface ou présentent une structure de surface physique.

11. Unité de test (100) selon l'une quelconque des revendications 1 à 10, dans laquelle la forme de section du deuxième évidement (114), dans une direction de vue généralement perpendiculaire à la direction d'écoulement du fluide corporel, s'élargit, en particulier s'élargit en continu, le long d'un trajet d'écoulement du fluide corporel le long du deuxième évidement (114).

12. Unité de test (100) selon l'une quelconque des revendications 1 à 11, dans laquelle au moins l'élément capillaire (110) est un composant moulé par injection.

13. Unité de test (100) selon l'une quelconque des revendications 1 à 12, dans laquelle l'élément capillaire (110) présente un bord tranchant (124) à son extrémité juxtaposant l'élément de test (108).

14. Unité de test (100) selon la revendication 13, dans laquelle le bord tranchant (124) présente l'un parmi le groupe constitué d'une forme en "V", d'une forme en "L" et d'une courbure de rayon de moins de 0,1 mm, en particulier de moins de 0,05 mm.

15. Unité de test (100) selon l'une quelconque des revendications 1 à 14, dans laquelle l'élément capillaire (110) présente une saillie (126) à son extrémité juxtaposant la surface de réception de partie de corps (102), dans laquelle le premier évidement (112) et le deuxième évidement (114) s'étendent à travers la saillie (126).

16. Unité de test (100) selon la revendication 15, dans laquelle le premier évidement (112) s'étend à travers une partie centrale de la saillie (126) et le deuxième évidement (114) s'étend à travers une partie latérale de la saillie (126).

17. Unité de test (100) selon la revendication 15 ou 16, dans laquelle une surface supérieure de la saillie (126) est inclinée de sorte que le deuxième évidement (114) s'étende à travers une partie de la saillie (126) à une extrémité supérieure de la surface supérieure inclinée.

18. Unité de test (100) selon l'une quelconque des revendications 15 à 17, dans laquelle une surface supérieure de la saillie (126) présente deux pointes entre lesquelles le deuxième évidement (114) s'étend à travers la saillie (126).

19. Unité de test (100) selon l'une quelconque des revendications 1 à 18, comprenant un capuchon (200), en particulier formé de manière solidaire avec un élément de corps (202) de l'unité de test (100), plus particulièrement connecté de manière pivotante à un élément de corps (202) de l'unité de test (100) par une charnière solidaire (204), le capuchon (200) étant configuré de manière à être ouvert sélectivement pour exposer la surface de réception de partie de corps (102) ou fermé pour recouvrir la surface de réception de partie de corps (102).

20. Unité de test (100) selon la revendication 19, comprenant au moins un trou de ventilation (206) destiné à créer une communication d'air entre la surface de réception de partie de corps (102) à recouvrir par le capuchon (200) et une autre partie de l'unité de test (100) étant exposée en permanence à la pression atmosphérique.

21. Unité de test (100) selon l'une quelconque des revendications 1 à 20, comprenant un élément de corps (202) et un élément de support (208) déplaçable par rapport à l'élément de corps (202) entre différentes positions, l'élément de support (208) recevant la lancette (104).

22. Unité de test (100) selon la revendication 21, dans laquelle l'élément de support (208) comprend un segment de fixation de dispositif de commande (210) configuré pour être fixé à un moyen de réception d'unité de test (2502) d'un dispositif de commande (2500).

23. Unité de test (100) selon l'une quelconque des revendications 1 à 22, comprenant une coupelle de pression (130) destinée à exercer une pression sur la partie de corps (118) pour favoriser l'écoulement de fluide hors d'un site à lancette et vers le deuxième évidement (114) de l'élément capillaire (110).

24. Unité de test (100) selon la revendication 23, dans laquelle la coupelle de pression (130) comprend un rebord circonférentiel qui entoure une extrémité supérieure, en particulier une saillie supérieure (126), de l'élément capillaire (110).

25. Unité de test (100) selon les revendications 21 et 23, dans laquelle l'unité de test (100) est configurée de sorte que le fait d'exercer une pression sur la coupelle de pression (130) déplace simultanément l'élément de support (208) par rapport à l'élément de corps (202) pour déplacer ainsi la lancette (104) de sorte que la pointe de lancette (106) perce la peau de la partie de corps (118) au moment de l'exercice d'une pression sur la coupelle de pression (130).

26. Unité de test (100) selon la revendication 25, dans laquelle l'unité de test (100) est configurée de sorte que la lancette (104) soit davantage déplacée pour être logée dans un état inactif à l'intérieur de l'unité de test (100) lors de l'exercice continué d'une pression après le perçage.

27. Unité de test (100) selon l'une quelconque des revendications 1 à 26, configurée comme une unité de test jetable autonome (100) qui ne peut être utilisée qu'une seule fois.

28. Unité de test (100) selon la revendication 1, dans laquelle l'élément de test (108) est une bague transparente qui comprend une substance qui est configurée pour réagir chimiquement avec le fluide corporel.

29. Unité de test (100) selon la revendication 28, dans laquelle l'élément de test (108) est configuré pour réagir chimiquement avec le fluide corporel pour ainsi créer un produit de réaction pouvant être détecté, en particulier détecté quantitativement, de manière photométrique.

30. Unité de test (100) selon la revendication 29, comprenant un élément optique (500), en particulier un guide de lumière, configuré pour manipuler optiquement, en particulier pour guider, un rayonnement électromagnétique d'au moins l'un parmi l'élément de test (108) et un élément d'étalonnage (502) vers une unité de détection d'un dispositif de commande (2500) sur lequel est reçue l'unité de test (100).

31. Unité de test (100) selon l'une quelconque des revendications 1 à 30, comprenant un élément d'étalonnage (502) configuré pour contenir des informations détectables pour l'étalonnage de l'unité de test (100).

32. Unité de test (100) selon la revendication 31, dans laquelle l'élément d'étalonnage (502) est une bague présentant un évidement central (504) pouvant être traversé par la lancette (104) et contenant les informations sous forme de marqueurs (506, 508) disposés de manière circonférentielle autour de l'évidement central (504).

33. Unité de test (100) selon la revendication 32, dans laquelle les marqueurs comprennent au moins l'un parmi le groupe constitué d'un code à barres (508) et au moins un champ de couleur de référence (506).

34. Unité de test (100) selon l'une quelconque des revendications 1 à 33, dans laquelle un volume du deuxième évidement (114) est de moins de 400 nl, en particulier de moins de 200 nl.

35. Appareil (2550) pour tester un fluide corporel, l'aménagement (2550) comprenant:
une unité de test autonome (100) selon l'une quelconque des revendications 1 à 34;
un dispositif de commande (2500) présentant:
un moyen de réception d'unité de test (2502) configuré pour recevoir de manière amovible l'unité de test (100);
une unité de détection configurée pour détecter les informations sur les composants de fluide corporel en déterminant un impact de l'interaction entre le fluide corporel et l'élément de test (108).

36. Appareil (2550) selon la revendication 35, dans lequel l'unité de détection est une unité de détection de rayonnement électromagnétique, en particulier une caméra numérique, plus particulièrement une caméra CMOS ou une caméra CCD, configurée pour détecter le rayonnement électromagnétique qui se propage de l'élément de test (108) vers l'unité de détection.

37. Appareil (2550) selon la revendication 35 ou 36, dans lequel le dispositif de commande (2500) est configuré comme un dispositif portable, par exemple comme un téléphone cellulaire.

38. Procédé de test de fluide corporel à l'aide d'une unité de test autonome (100), le procédé comprenant le fait de:
recevoir la partie de corps d'un patient (118) sur une surface de réception de partie de corps (102) de l'unité de test (100);
déplacer une lancette (104), présentant une pointe de lancette (106), de l'unité de test (100) à travers un premier évidement (112) d'un élément capillaire (110) de l'unité de test (100) de sorte que la pointe de lancette (106) soit en interaction avec la partie de corps du patient (118) pour percer la peau du patient pour produire un écoulement de liquide corporel;
amener un élément de test (108) de l'unité de test (100) en interaction avec le fluide corporel en guidant le fluide corporel de la surface de réception de partie de corps (102) à travers un deuxième évidement (114) de l'élément capillaire (110) vers l'élément de test (108) pour aider à déterminer les informations sur les composants de fluide corporel;
dans lequel le deuxième évidement (114) présente un rapport superficie/volume plus grand que le premier évidement (112), dans lequel l'élément de test (108) est configuré pour interagir avec le fluide corporel pour aider à déterminer les informations sur les composants de fluide corporel, et l'élément de test (108) présente un évidement central (504) pouvant être traversé par la lancette (104).
